# EUROPEAN PATENT APPLICATION

(11) **EP 3 460 057 A1**
(43) Date of publication of application: **27.03.2019**
(21) Application number: 17192670.2
(22) Date of filing: 22.09.2017
(51) Int. Cl.: C12N 9/80

(54) **KDAC VARIANTS AND USES THEREOF**

(71) Applicant: Max-Planck-Gesellschaft zur Förderung der Wissenschaften e.V., 80539 München (DE)
(72) Inventor: NEUMANN, Heinz, 44149 Dortmund (DE); SPINCK, Martin, 44149 Dortmund (DE)
(74) Representative: Ricker, Mathias

(57) **Abstract**

The present invention relates to a mutant polypeptide comprising an amino acid sequence having at least 98% sequence homology with SEQ ID NOs: 4, 5, 6, 7 or 8 and having lysine demodification activity, wherein the mutant polypeptide is not identical to SEQ ID NO: 1. The invention also relates to the mutant polypeptide of the invention and a peptide or polypeptide comprising an inactivated essential lysine residue for use in treating cancer. The invention furthermore provides for a method of screening for a mutant polypeptide having lysine demodification activity, wherein the method comprises the following steps (a) incubating a mutant polypeptide having an amino acid sequence with at least 80% sequence identity to SEQ ID NO: 1 with a peptide or polypeptide comprising an inactivated essential lysine residue; and (b) determining the activity of the mutant polypeptide to activate the peptide or polypeptide comprising the inactivated essential lysine residue, wherein the mutant polypeptide and the peptide or polypeptide comprising an inactivated essential lysine residue are incubated in a biological cell.

## Description

The present invention relates to a mutant polypeptide comprising an amino acid sequence having at least 98% sequence homology with SEQ ID NOs: 4, 5, 6, 7 or 8 and having lysine demodification activity, wherein the mutant polypeptide is not identical to SEQ ID NO: 1. The invention also relates to the mutant polypeptide of the invention and a peptide or polypeptide comprising an inactivated essential lysine residue for use in treating cancer. The invention furthermore provides for a method of screening for a mutant polypeptide having lysine demodification activity, wherein the method comprises the following steps (a) incubating a mutant polypeptide having an amino acid sequence with at least 80% sequence identity to SEQ ID NO: 1 with a peptide or polypeptide comprising an inactivated essential lysine residue; and (b) determining the activity of the mutant polypeptide to activate the peptide or polypeptide comprising the inactivated essential lysine residue, wherein the mutant polypeptide and the peptide or polypeptide comprising an inactivated essential lysine residue are incubated in a biological cell.

Lysine Deacetylases (KDACs) are a prominent class of enzymes featuring roles in almost all physiological processes and many diseases including cancer and aging. These enzymes reverse various types of lysine acylations thereby controlling, e.g., enzyme activities, protein localization and chromatin structure. Acetylation of the N(ε)-amino group of lysine residues was initially discovered fifty years ago on histone proteins. The past two decades revealed a large variety of functional roles of this modification in almost every physiological process. The spectrum of acylation found on lysine side chains is not restricted to acetylation but broad, ranging from short acyl chains to fatty acids and charged functional groups. All these modifications are reversed by a comparably small set of lysine deacetylases (KDACs), which are categorized in four enzyme families. The related class 1, 2 and 4 enzymes are structurally and mechanistically distinct from class 3 KDACs. The formers contain a zinc ion in the active site to orient a water molecule and polarize the substrate, while the latter use NAD+ as a co-substrate to cleave the amide bond. KDACs feature prominently in many physiological processes. Initially discovered on histones, they are well-known as repressors of transcription because removal of the acyl groups enhances histone-DNA contacts and hence leads to chromatin compaction. The discovery of thousands of acylation sites in different organisms from all kingdoms of life gives us an idea of the importance of this modification for the regulation of cellular processes. Defects in these enzymes are connected to a variety of diseases such as diabetes, cancer and aging. Exactly how KDAC misregulation contributes to disease etiology is often difficult to trace because of the limited specificity of the enzymes for particular protein substrates and types of acylation. Genetic ablation of KDACs causes pleiotropic effects mediated by altered gene expression levels. KDAC inhibitors are valuable tools in functional studies and active leads in pharmaceutical design. Unfortunately, their selectivity for particular KDACs is limited, making the interpretation of results more difficult and restricting clinical use.

KDAC variants selective for particular types of lysine modifications would be highly useful. Moreover, there is current need in the art for improved cancer therapies, which cause less severe side-effects and which are highly selective in terms of site of action and time.

The technical problem underlying the present invention is thus the provision of novel KDAC variants with improved activity towards the removal of lysine modifications, methods of screening for KDAC variants and uses thereof.

The technical problem is solved by the embodiments as defined in the claims.

The present invention thus relates to a mutant polypeptide comprising an amino acid sequence having at least 98% sequence homology with SEQ ID NOs: 4, 5, 6, 7 or 8 and having lysine demodification activity, wherein the mutant polypeptide is not identical to SEQ ID NO: 1. The invention also relates to a mutant polypeptide, wherein the mutant polypeptide comprises an amino acid sequence having 1 to 5 mutations in the amino acid sequence of SEQ ID NO: 1.

As shown in the appended examples, the KDAC variants of the invention surprisingly and unexpectedly remove typical protection groups for lysine side chains to an extent sufficient to activate an amount of Ura3 enzyme to sustain cell growth of bacterial cells in the absence of uracil. Such an activity is surprising and unexpected in view of the prior art, which has been unable to provide KDAC variants showing such an improved activity, which allows bacterial cells to grow in the absence of essential growth medium components such as uracil. The mutant polypeptides of the invention, catalysing bioorthogonal reactions are the key to success for save prodrug strategies in cancer therapy. Presently, enzymes to activate prodrugs are either of human origin (with the disadvantage of being present in other tissues and therefore causing side effects) or from a different organism (with the disadvantage of being immunogenic). The mutant polypeptides of the invention with bioorthogonal activity evolved from a parent enzyme of human origin combine the advantages of both approaches.

In one embodiment of the invention, the mutant polypeptide comprises a mutation of A37S, Y53W, R56W, I53V and/or V148L with respect to SEQ ID NO: 1. These mutations have been shown to surprisingly and unexpectedly significantly improve the activity of KDAC to an extent as shown herein.

The mutant polypeptide of the invention preferably comprises a sequence identical to any one of SEQ ID NOs: 4, 5, 6, 7 or 8. More preferably, the mutant polypeptide of the invention is identical to any one of SEQ ID NOs: 4, 5, 6, 7 or 8.

Accordingly, the present invention is not restricted to KDAC variants as in any one of SEQ ID NOS: 4, 5, 6, 7 or 8, but extends, in particular, to KDAC variants which are structurally related to any of the above variants such as, e.g., truncated versions thereof. Thus, the present invention also relates to variants of KDAC, which are structurally related to KDAC variants as in any one of SEQ ID NOS: 4, 5, 6, 7 or 8 and which show one or more substitutions and/or deletions and/or insertions. The term "structurally related" refers to KDAC variants, which show a sequence identity of at least n% to the sequence shown in any one of SEQ ID NOS: 4, 5, 6, 7 or 8 with n being between 98 and 100, but not identical to SEQ ID NO: 1.

Thus, in one embodiment the variant according to the present invention has or preferably is derived from a sequence which is at least n % identical to any one of SEQ ID NOS: 4, 5, 6, 7 or 8 with n being between 98 and 100, and it has (a) substitution(s) and/or (a) deletion and/or (an) insertion(s). When the sequences which are compared do not have the same length, the degree of identity either refers to the percentage of amino acid residues in the shorter sequence which are identical to amino acid residues in the longer sequence or to the percentage of amino acid residues in the longer sequence which are identical to amino acid residues in the shorter sequence. Preferably, it refers to the percentage of amino acid residues in the shorter sequence, which are identical to amino acid residues in the longer sequence. The degree of sequence identity can be determined according to methods well known in the art using preferably suitable computer algorithms such as CLUSTAL.

When using the Clustal analysis method to determine whether a particular sequence is, for instance, at least 98% identical to a reference sequence default settings may be used or the settings are preferably as follows: Matrix: blosum 30; Open gap penalty: 10.0; Extend gap penalty: 0.05; Delay divergent: 40; Gap separation distance: 8 for comparisons of amino acid sequences. For nucleotide sequence comparisons, the Extend gap penalty is preferably set to 5.0.
In a preferred embodiment ClustalW2 is used for the comparison of amino acid sequences. In the case of pairwise comparisons/alignments, the following settings are preferably chosen: Protein weight matrix: BLOSUM 62; gap open: 10; gap extension: 0.1. In the case of multiple comparisons/alignments, the following settings are preferably chosen: Protein weight matrix: BLOSUM 62; gap open: 10; gap extension: 0.2; gap distance: 5; no end gap.
Preferably, the degree of identity is calculated over the complete length of the sequence. Amino acid residues located at a position corresponding to a position as indicated hereinbelow in the amino acid sequence shown in any one of SEQ ID NOS: 4, 5, 6, 7 or 8 can be identified by the skilled person by methods known in the art. For example, such amino acid residues can be identified by aligning the sequence in question with the sequence shown in SEQ ID NO:1 and by identifying the positions which correspond to the above indicated positions of SEQ ID NO:1. The alignment can be done with means and methods known to the skilled person, e.g. by using a known computer algorithm such as the Lipman-Pearson method (Science 227 (1985), 1435) or the CLUSTAL algorithm. It is preferred that in such an alignment maximum homology is assigned to conserved amino acid residues present in the amino acid sequences.
In a preferred embodiment ClustalW2 is used for the comparison of amino acid sequences. In the case of pairwise comparisons/alignments, the following settings are preferably chosen: Protein weight matrix: BLOSUM 62; gap open: 10; gap extension: 0.1. In the case of multiple comparisons/alignments, the following settings are preferably chosen: Protein weight matrix: BLOSUM 62; gap open: 10; gap extension: 0.2; gap distance: 5; no end gap.

When the amino acid sequences of the mutant polypeptides are aligned by means of such a method, regardless of insertions or deletions that occur in the amino acid sequences, the positions of the corresponding amino acid residues can be determined in each of the KDAC variants.
In the context of the present invention, "substituted with another amino acid residue" means that the respective amino acid residues at the indicated position can be substituted with any other possible amino acid residues, e.g. naturally occurring amino acids or non-naturally occurring amino acids (Brustad and Arnold, Curr. Opin. Chem. Biol. 15 (2011), 201-210), preferably with an amino acid residues selected from the group consisting of alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine and valine. Preferred substitutions for certain positions are indicated further below. Moreover, the term "substituted" or "substitution" also means that the respective amino acid residue at the indicated position is modified.
Such modifications include naturally occurring modifications and non-naturally occurring modifications. Naturally occurring modifications include but are not limited to eukaryotic post-translational modification, such as attachment of functional groups (e.g. acetate, phosphate, hydroxyl, lipids (myristoylation of glycine residues) and carbohydrates (e.g. glycosylation of arginine, asparagines etc.). Naturally occurring modifications also encompass the change in the chemical structure by citrullination, carbamylation and disulphide bond formation between cysteine residues; attachment of co-factors (FMN or FAD that can be covalently attached) or the attachment of peptides (e.g. ubiquitination or sumoylation).
Non-naturally occurring modifications include, e.g., in vitro modifications such as biotinylation of lysine residue or the inclusion of non-canonical amino acids (see Liu and Schultz, Annu. Rev. Biochem. 79 (2010), 413-44 and Wang et al., Chem. Bio. 2009 March 27; 16 (3), 323-336; doi:101016/jchembiol.2009.03.001).
In the context of the present invention, "deleted" or "deletion" means that the amino acid at the corresponding position is deleted.
In the context of the present invention, "inserted" or "insertion" means that at the respective position one or two, preferably one amino acid residue is inserted, preferably in front of the indicated position.

In accordance with the foregoing, the present invention relates to a variant of KDAC, wherein the KDAC variant is characterized in that it shows one or more substitutions, deletions and/or insertions in comparison to the corresponding sequence from which it is derived and wherein these substitutions, deletions and/or insertions occur at one or more of the positions corresponding to positions 37, 53, 56, 92 and/or 148 in the amino acid sequence shown in SEQ ID NO:1. Thus, in one embodiment, the invention relates to a mutant polypeptide having a sequence of SEQ ID NO:1 with 1 to 5 amino acid substitutions, preferably at positions 37, 53, 56, 92 and/or 148 and more preferably mutations A37S, Y53W, R56W, I92V and/or V148L.

In a preferred embodiment, the variant according to the invention is characterized in that it contains at least two deletions, substitutions and/or insertions wherein the deletion/insertion/substitution is at position 37 in the amino acid sequence shown in SEQ ID NO: 1 or at a position corresponding to this position and another deletion/insertion/substitution is at position 53 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 56, 92 and/or 148 in the amino acid sequence shown in SEQ ID NO:1.
In a preferred embodiment, the variant according to the invention is characterized in that it contains at least two deletions, substitutions and/or insertions wherein the deletion/insertion/substitution is at position 37 in the amino acid sequence shown in SEQ ID NO:1 or at a position corresponding to this position and another deletion/insertion/substitution is at position 56 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 53, 92 and/or 148 in the amino acid sequence shown in SEQ ID NO:1.
In a preferred embodiment, the variant according to the invention is characterized in that it contains at least two deletions, substitutions and/or insertions wherein the deletion/insertion/substitution is at position 37 in the amino acid sequence shown in SEQ ID NO: 1 or at a position corresponding to this position and another deletion/insertion/substitution is at position 92 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 53, 56 and/or 148 in the amino acid sequence shown in SEQ ID NO:1.
In a preferred embodiment, the variant according to the invention is characterized in that it contains at least two deletions, substitutions and/or insertions wherein the deletion/insertion/substitution is at position 37 in the amino acid sequence shown in SEQ ID NO: 1 or at a position corresponding to this position and another deletion/insertion/substitution is at position 148 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 53, 56 and/or 92 in the amino acid sequence shown in SEQ ID NO:1.
In a preferred embodiment, the variant according to the invention is characterized in that it contains at least two deletions, substitutions and/or insertions wherein the deletion/insertion/substitution is at position 53 in the amino acid sequence shown in SEQ ID NO: 1 or at a position corresponding to this position and another deletion/insertion/substitution is at position 56 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 37, 92 and/or 148 in the amino acid sequence shown in SEQ ID NO:1.
In a preferred embodiment, the variant according to the invention is characterized in that it contains at least two deletions, substitutions and/or insertions wherein the deletion/insertion/substitution is at position 53 in the amino acid sequence shown in SEQ ID NO: 1 or at a position corresponding to this position and another deletion/insertion/substitution is at position 92 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 37, 56 and/or 148 in the amino acid sequence shown in SEQ ID NO:1.
In a preferred embodiment, the variant according to the invention is characterized in that it contains at least two deletions, substitutions and/or insertions wherein the deletion/insertion/substitution is at position 53 in the amino acid sequence shown in SEQ ID NO: 1 or at a position corresponding to this position and another deletion/insertion/substitution is at position 148 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 37, 56 and/or 92 in the amino acid sequence shown in SEQ ID NO:1.
In a preferred embodiment, the variant according to the invention is characterized in that it contains at least two deletions, substitutions and/or insertions wherein the deletion/insertion/substitution is at position 56 in the amino acid sequence shown in SEQ ID NO: 1 or at a position corresponding to this position and another deletion/insertion/substitution is at position 92 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 37, 53 and/or 148 in the amino acid sequence shown in SEQ ID NO:1.
In a preferred embodiment, the variant according to the invention is characterized in that it contains at least two deletions, substitutions and/or insertions wherein the deletion/insertion/substitution is at position 56 in the amino acid sequence shown in SEQ ID NO: 1 or at a position corresponding to this position and another deletion/insertion/substitution is at position 148 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 37, 53 and/or 92 in the amino acid sequence shown in SEQ ID NO:1.
In a preferred embodiment, the variant according to the invention is characterized in that it contains at least two deletions, substitutions and/or insertions wherein the deletion/insertion/substitution is at position 92 in the amino acid sequence shown in SEQ ID NO: 1 or at a position corresponding to this position and another deletion/insertion/substitution is at position 148 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 37, 53 and/or 56 in the amino acid sequence shown in SEQ ID NO:1.
In a preferred embodiment, the variant according to the invention is characterized in that it contains at least three deletions, substitutions and/or insertions wherein the deletion/insertion/substitution is at position 37 in the amino acid sequence shown in SEQ ID NO:1 or at a position corresponding to this position and another deletion/insertion/substitution is at position 53 in the amino acid sequence shown in SEQ ID NO:1 or at a position corresponding to this position and another deletion/insertion/substitution is at position 56 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 92 and/or 148 in the amino acid sequence shown in SEQ ID NO:1.
In a preferred embodiment, the variant according to the invention is characterized in that it contains at least three deletions, substitutions and/or insertions wherein the deletion/insertion/substitution is at position 37 in the amino acid sequence shown in SEQ ID NO:1 or at a position corresponding to this position and another deletion/insertion/substitution is at position 53 in the amino acid sequence shown in SEQ ID NO:1 or at a position corresponding to this position and another deletion/insertion/substitution is at position 92 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 56 and/or 148 in the amino acid sequence shown in SEQ ID NO:1.
In a preferred embodiment, the variant according to the invention is characterized in that it contains at least three deletions, substitutions and/or insertions wherein the deletion/insertion/substitution is at position 37 in the amino acid sequence shown in SEQ ID NO:1 or at a position corresponding to this position and another deletion/insertion/substitution is at position 53 in the amino acid sequence shown in SEQ ID NO:1 or at a position corresponding to this position and another deletion/insertion/substitution is at position 148 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 53 and/or 92 in the amino acid sequence shown in SEQ ID NO:1.
In a preferred embodiment, the variant according to the invention is characterized in that it contains at least three deletions, substitutions and/or insertions wherein the deletion/insertion/substitution is at position 37 in the amino acid sequence shown in SEQ ID NO:1 or at a position corresponding to this position and another deletion/insertion/substitution is at position 56 in the amino acid sequence shown in SEQ ID NO:1 or at a position corresponding to this position and another deletion/insertion/substitution is at position 92 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 53 and/or 148 in the amino acid sequence shown in SEQ ID NO:1.
In a preferred embodiment, the variant according to the invention is characterized in that it contains at least three deletions, substitutions and/or insertions wherein the deletion/insertion/substitution is at position 37 in the amino acid sequence shown in SEQ ID NO:1 or at a position corresponding to this position and another deletion/insertion/substitution is at position 56 in the amino acid sequence shown in SEQ ID NO:1 or at a position corresponding to this position and another deletion/insertion/substitution is at position 148 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 53 and/or 92 in the amino acid sequence shown in SEQ ID NO:1.

In a preferred embodiment, the variant according to the invention is characterized in that it contains at least three deletions, substitutions and/or insertions wherein the deletion/insertion/substitution is at position 53 in the amino acid sequence shown in SEQ ID NO:1 or at a position corresponding to this position and another deletion/insertion/substitution is at position 56 in the amino acid sequence shown in SEQ ID NO:1 or at a position corresponding to this position and another deletion/insertion/substitution is at position 92 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 37 and/or 148 in the amino acid sequence shown in SEQ ID NO:1.
In a preferred embodiment, the variant according to the invention is characterized in that it contains at least three deletions, substitutions and/or insertions wherein the deletion/insertion/substitution is at position 53 in the amino acid sequence shown in SEQ ID NO:1 or at a position corresponding to this position and another deletion/insertion/substitution is at position 56 in the amino acid sequence shown in SEQ ID NO:1 or at a position corresponding to this position and another deletion/insertion/substitution is at position 148 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 37 and/or 92 in the amino acid sequence shown in SEQ ID NO:1.
In a preferred embodiment, the variant according to the invention is characterized in that it contains at least three deletions, substitutions and/or insertions wherein the deletion/insertion/substitution is at position 56 in the amino acid sequence shown in SEQ ID NO:1 or at a position corresponding to this position and another deletion/insertion/substitution is at position 92 in the amino acid sequence shown in SEQ ID NO:1 or at a position corresponding to this position and another deletion/insertion/substitution is at position 148 in the amino acid sequence shown in SEQ ID NO:1 or at a position corresponding to this position. Preferably, such a variant further has one or more substitutions, deletions and/or insertions in comparison to the corresponding sequence from which it is derived and wherein these substitutions, deletions and/or insertions occur at one or more of the positions corresponding to positions 37 and/or 53 in the amino acid sequence shown in SEQ ID NO:1.
In a preferred embodiment, the variant according to the invention is characterized in that it contains at least four deletions, substitutions and/or insertions wherein the deletion/insertion/substitution is at position 37 in the amino acid sequence shown in SEQ ID NO:1 or at a position corresponding to this position and another deletion/insertion/substitution is at position 53 in the amino acid sequence shown in SEQ ID NO:1 or at a position corresponding to this position and another deletion/insertion/substitution is at position 56 in the amino acid sequence shown in SEQ ID NO:1 or at a position corresponding to this position and another deletion/insertion/substitution is at position 92 in the amino acid sequence shown in SEQ ID NO:1 or at a position corresponding to this position. Preferably, such a variant further has one substitution, deletion and/or insertion in comparison to the corresponding sequence from which it is derived and wherein this substitution, deletion and/or insertion occurs at the position corresponding to position 148 in the amino acid sequence shown in SEQ ID NO:1.
In a preferred embodiment, the variant according to the invention is characterized in that it contains at least four deletions, substitutions and/or insertions wherein the deletion/insertion/substitution is at position 37 in the amino acid sequence shown in SEQ ID NO:1 or at a position corresponding to this position and another deletion/insertion/substitution is at position 53 in the amino acid sequence shown in SEQ ID NO:1 or at a position corresponding to this position and another deletion/insertion/substitution is at position 56 in the amino acid sequence shown in SEQ ID NO:1 or at a position corresponding to this position and another deletion/insertion/substitution is at position 148 in the amino acid sequence shown in SEQ ID NO:1 or at a position corresponding to this position. Preferably, such a variant further has one substitution, deletion and/or insertion in comparison to the corresponding sequence from which it is derived and wherein this substitution, deletion and/or insertion occurs at the position corresponding to position 92 in the amino acid sequence shown in SEQ ID NO:1.
In a preferred embodiment, the variant according to the invention is characterized in that it contains at least four deletions, substitutions and/or insertions wherein the deletion/insertion/substitution is at position 53 in the amino acid sequence shown in SEQ ID NO:1 or at a position corresponding to this position and another deletion/insertion/substitution is at position 56 in the amino acid sequence shown in SEQ ID NO:1 or at a position corresponding to this position and another deletion/insertion/substitution is at position 92 in the amino acid sequence shown in SEQ ID NO:1 or at a position corresponding to this position and another deletion/insertion/substitution is at position 148 in the amino acid sequence shown in SEQ ID NO:1 or at a position corresponding to this position. Preferably, such a variant further has one substitution, deletion and/or insertion in comparison to the corresponding sequence from which it is derived and wherein this substitution, deletion and/or insertion occurs at the position corresponding to position 37 in the amino acid sequence shown in SEQ ID NO:1.

In even more preferred embodiments, the variant according to the invention showing an improved activity in demodification of an essential lysine residue is characterized in that it has multiple mutations. As it is exemplified in the examples further below, variants have been found bearing multiple mutations which exhibit an increase in the reaction rate of the conversion of a modified essential lysine residue to the unmodified lysine. These variants bearing multiple mutations are summarized in the following. Accordingly, in a very preferred embodiment, the variant according to the invention is characterized in that it comprises deletions, substitutions and/or insertions wherein the deletions/insertions/substitutions are at positions 37, 53, 56, 92 and 148 in the amino acid sequence shown in SEQ ID NO:1 or at positions corresponding to these positions. Preferably, such a variant has the following substitutions in the amino acid sequence shown in SEQ ID NO:1 or at positions corresponding to these positions:
A37S, Y53W, R56W, I92V and V148L.

In another preferred embodiment, the variant according to the invention is characterized in that it comprises deletions, substitutions and/or insertions wherein the deletions/insertions/substitutions are at positions 37, 56 and 92 in the amino acid sequence shown in SEQ ID NO:1 or at positions corresponding to these positions. Preferably, such a variant has the following substitutions in the amino acid sequence shown in SEQ ID NO:1 or at positions corresponding to these positions:
A37S, R56G and I92V.

In another preferred embodiment, the variant according to the invention is characterized in that it comprises deletions, substitutions and/or insertions wherein the deletions/insertions/substitutions are at positions 92 and 148 in the amino acid sequence shown in SEQ ID NO:1 or at positions corresponding to these positions. Preferably, such a variant has the following substitutions in the amino acid sequence shown in SEQ ID NO:1 or at positions corresponding to these positions:
192S and V148L.

In another preferred embodiment, the variant according to the invention is characterized in that it comprises deletions, substitutions and/or insertions wherein the deletions/insertions/substitutions are at positions 92 and 148 in the amino acid sequence shown in SEQ ID NO:1 or at positions corresponding to these positions. Preferably, such a variant has the following substitutions in the amino acid sequence shown in SEQ ID NO:1 or at positions corresponding to these positions:
192V and V148L.

In another preferred embodiment, the variant according to the invention is characterized in that it comprises deletions, substitutions and/or insertions wherein the deletions/insertions/substitutions are at positions 37, 53, 56, 92 and 148 in the amino acid sequence shown in SEQ ID NO:1 or at positions corresponding to these positions. Preferably, such a variant has the following substitutions in the amino acid sequence shown in SEQ ID NO:1 or at positions corresponding to these positions:
A37R, Y53G, R56T, I92R and V148L.

Conservative substitutions of peptides/polypeptides, which may furthermore be part of the mutant polypeptides of the invention, are shown below.
Ala (A) Val; Leu;
Arg (R) Lys; His
Asn (N) Gln; His; Asp, Lys; Arg
Asp (D) Glu; Asn
Cys (C) Ser; Ala
Gln (Q) Asn; Glu
Glu (E) Asp; Gln
Gly (G) Ala
His (H) Asn; Gln; Lys; Arg
He (I) Leu; Val; Met; Ala; Phe; Norleucine
Leu (L) Norleucine; Ile; Val; Met; Ala; Phe
Lys (K) Arg; Gln; Asn
Met (M) Leu; Phe; Ile
Phe (F) Trp; Leu; Val; Ile; Ala; Tyr
Pro (P) Ala
Ser (S) Thr
Thr (T) Val; Ser
Trp (W) Tyr; Phe
Tyr (Y) Trp; Phe; Thr; Ser
Val (V) Ile; Leu; Met; Phe; Ala; Norleucine

Amino acids may be grouped according to common side-chain properties:
(1) hydrophobic: Norleucine, Met, Ala, Val, Leu, Ile
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Amino acids may also be grouped according to common side-chain size, for example, small amino acids (Gly, Ala, Ser, Pro, Thr, Asp, Asn), or bulky hydrophobic amino acids (Met, Ile, Leu). Substantial modifications in the biological properties of the peptide/polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Non-conservative substitutions will entail exchanging a member of one of these classes for another class.

In further embodiments, peptides or polypeptides of the invention may comprise one or more non-naturally occurring or modified amino acids. A "non-naturally occurring amino acid residue" refers to a residue, other than those naturally occurring amino acid residues listed above, which is able to covalently bind adjacent amino acid residues(s) in a polypeptide chain. Non-natural amino acids include, but are not limited to, homo-lysine, homo-arginine, homo-serine, azetidinecarboxylic acid, 2-aminoadipic acid, 3-aminoadipic acid, beta-alanine, aminopropionic acid, 2-aminobutyric acid, 4-aminobutyric acid, 6-aminocaproic acid, 2- aminoheptanoic acid, 2-aminoisobutyric acid, 3-aminoisobutyric acid, 2-aminopimelic acid, tertiary-butylglycine, 2,4-diaminoisobutyric acid, desmosine, 2,2'-diaminopimelic acid, 2,3-diaminopropionic acid, N-ethylglycine, N-ethylasparagine, homoproline, hydroxylysine, allo-hydroxylysine, 3-hydroxyproline, 4-hydroxyproline, isodesmosine, allo-isoleucine, N-methylalanine, N-methylglycine, N-methylisoleucine, N-methylpentylglycine, N-methylvaline, naphthalanine, norvaline, norleucine, ornithine, citrulline, pentylglycine, pipecolic acid and thioproline. Modified amino acids include natural and non-natural amino acids which are chemically blocked, reversibly or irreversibly, or modified on their N-terminal amino group or their side chain groups, as for example, N-methylated D and L amino acids, side chain functional groups that are chemically modified to another functional group. For example, modified amino acids include methionine sulfoxide; methionine sulfone; aspartic acid- (beta-methyl ester), a modified amino acid of aspartic acid; N-ethylglycine, a modified amino acid of glycine; or alanine carboxamide and a modified amino acid of alanine. Additional non-natural and modified amino acids, and methods of incorporating them into proteins and peptides, are known in the art (see, e.g., Sandberg et al. , (1998) J. Med. Chem. 41 : 2481-91; Xie and Schultz (2005) Curr. Opin. Chem. Biol. 9: 548-554; Hodgson and Sanderson (2004) Chem. Soc. Rev. 33: 422-430; Dumas et al. ChemSci (2014) 6(1), 50-69).

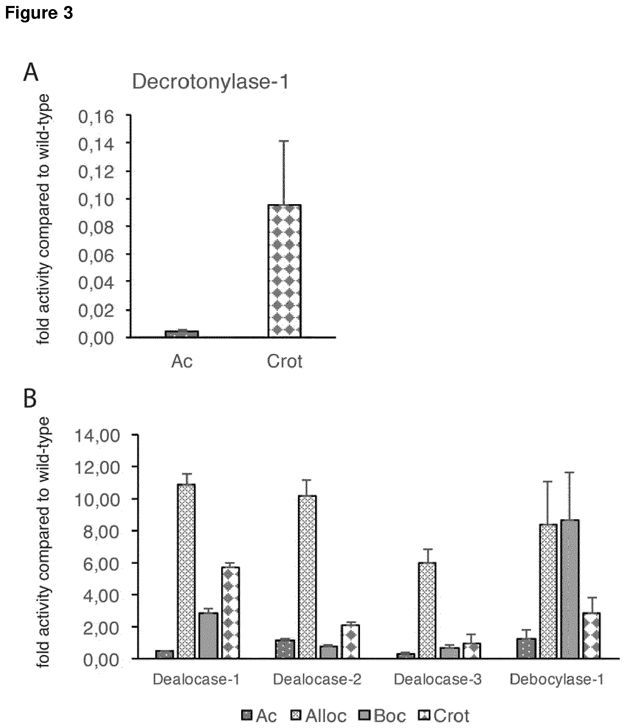

The KDAC variants of the invention have an improved activity of demodification of lysine as compared to the unmodified KDAC polypeptide as shown in SEQ ID NO: 1. In this respect, an "improved activity of demodification of lysine" or similar terms as used herein, can be determined by, for example, methods using a Firefly luciferase with modifications on lysine-529. Specifically, demodification activity can be determined using an assay where the KDAC variant of the invention is incubated with the modified Firefly luciferase directly in a whole cell lysate and activity is compared to the activity of wild-type KDAC, in particular cobB. Additionally or alternatively, activities of KDAC variants can be assayed using the bacterial system described further below. Both tests have been surprisingly and unexpectedly shown to provide comparable results (Figure 3).

In a further embodiment, the present invention relates to a nucleic acid molecule encoding the KDAC variant of the invention. Moreover, the present invention relates in a further embodiment to a vector comprising said nucleic acid. Further, in yet another embodiment, the present invention relates to a host cell comprising said vector. The embodiments relating to the nucleic acid, the vector and the host cell of the present invention are further described in the following in more detail.

A KDAC variant of the present invention can be fused to a homologous or heterologous polypeptide or protein, an enzyme, a substrate or a tag to form a fusion protein. Fusion proteins in accordance with the present invention will have the same improved activity as the KDAC variant of the present invention. Polypeptides, enzymes, substrates or tags that can be added to another protein are known in the art. They may be useful for purifying or detecting the proteins of the invention. For instance, tags that can be used for detection and/or purification are e.g. FLAG-tag, His6-tag or a Strep-tag. Alternatively, the protein of the invention can be fused to an enzyme e.g. luciferase, for the detection or localisation of said protein. Other fusion partners include, but are not limited to, bacterial β-galactosidase, trpE, Protein A, β-lactamase, alpha amylase, alcohol dehydrogenase or yeast alpha mating factor. It is also conceivable that the polypeptide, enzyme, substrate or tag is removed from the protein of the invention after e.g. purification. Fusion proteins can typically be made by either recombinant nucleic acid methods or by synthetic polypeptide methods known in art.

The present invention further relates to a nucleic acid molecule encoding a KDAC variant of the present invention and to a vector comprising said nucleic acid molecules. Vectors that can be used in accordance with the present invention are known in the art. The vectors can further comprise expression control sequences operably linked to the nucleic acid molecules of the present invention contained in the vectors. These expression control sequences may be suited to ensure transcription and synthesis of a translatable RNA in bacteria or fungi. Expression control sequences can for instance be promoters. Promoters for use in connection with the nucleic acid molecules of the present invention may be homologous or heterologous with regard to its origin and/or with regard to the gene to be expressed. Suitable promoters are for instance promoters which lend themselves to constitutive expression. However, promoters which are only activated at a point in time determined by external influences can also be used. Artificial and/or chemically inducible promoters may be used in this context.

Polynucleotide," or "nucleic acid," as used interchangeably herein, refer to polymers of nucleotides of any length, and include, but are not limited to, DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, cabamates, etc.) and with charged linkages (e.g., phosphorothioates, phosphorodithioates, etc.), those containing pendant moieties, such as, for example, proteins (e.g., nucleases, toxins, antibodies, signal peptides, poly-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping groups moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl, 2'-fluoro- or 2'-azido-ribose, carbocyclic sugar analogs, alpha.-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR.sub.2 ("amidate"), P(O)R, P(O)OR, CO or CH.sub.2 ("formacetal"), in which each R or R is independently H or substituted or unsubstituted alkyl (1-20 C.) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

The polynucleotide(s) of the present invention may be part of a vector. Preferably, the vector of the present invention is an expression vector. Expression vectors have been widely described in the literature. As a rule, they contain not only a selection marker gene and a replication-origin ensuring replication in the host selected, but also a bacterial or viral promoter, and in most cases a termination signal for transcription. Between the promoter and the termination signal there is in general at least one restriction site or a polylinker which enables the insertion of a coding DNA sequence. The DNA sequence naturally controlling the transcription of the corresponding gene can be used as the promoter sequence, if it is active in the selected host organism. However, this sequence can also be exchanged for other promoter sequences. It is possible to use promoters ensuring constitutive expression of the gene and inducible promoters which permit a deliberate control of the expression of the gene. Bacterial and viral promoter sequences possessing these properties are described in detail in the literature. Regulatory sequences for the expression in microorganisms (for instance *E*. *coli, S. cerevisiae)* are sufficiently described in the literature. Promoters permitting a particularly high expression of a downstream sequence are for instance the T7 promoter (Studier et al., Methods in Enzymology 185 (1990), 60-89), lacUV5, trp, trp-lacUV5 (DeBoer et al., in Rodriguez and Chamberlin (Eds), Promoters, Structure and Function; Praeger, New York, (1982), 462-481; DeBoer et al., Proc. Natl. Acad. Sci. USA (1983), 21-25), Ip1, rac (Boros et al., Gene 42 (1986), 97-100). Inducible promoters are preferably used for the synthesis of polypeptides. These promoters often lead to higher polypeptide yields than do constitutive promoters. In order to obtain an optimum amount of polypeptide, a two-stage process is often used. First, the host cells are cultured under optimum conditions up to a relatively high cell density. In the second step, transcription is induced depending on the type of promoter used. In this regard, a tac promoter is particularly suitable which can be induced by lactose or IPTG (=isopropyl-ß-D-thiogalactopyranoside) (deBoer et al., Proc. Natl. Acad. Sci. USA 80 (1983), 21-25). Termination signals for transcription are also described in the literature.

In addition, the present invention relates to a host cell comprising the vector of the present invention.
In a preferred embodiment, the host cell according to the presenting invention is a microorganism, in particular a bacterium or a fungus. In a more preferred embodiment, the host cell of the present invention is E. coli, a bacterium of the genus Clostridium or a yeast cell, such as S. cerevisiae. In another preferred embodiment the host cell is a plant cell or a non-human animal cell.
The transformation of the host cell with a vector according to the invention can be carried out by standard methods, as for instance described in Sambrook and Russell (2001), Molecular Cloning: A Laboratory Manual, CSH Press, Cold Spring Harbor, NY, USA; Methods in Yeast Genetics, A Laboratory Course Manual, Cold Spring Harbor Laboratory Press, 1990. The host cell is cultured in nutrient media meeting the requirements of the particular host cell used, in particular in respect of the pH value, temperature, salt concentration, aeration, antibiotics, vitamins, trace elements etc.

In one preferred embodiment, the organism according to the present invention which can be employed in the method according to the invention is an organism, preferably a microorganism, which lacks the capacity to produce an essentially required factor for growth. For example, the organism, preferably the microorganism, may lack the capacity to produce essential amino acid(s) or nucleobase(s). This is preferably achieved by deleting or otherwise modifying one or more enzymes necessary for the production of the said factor, e.g. enzymes converting precursors of such actors to the ultimately essential factor. One example within the meaning of the present invention is Ura3, which is necessary to produce uracil. The enzyme that is modified/inactivated carries an essential lysine residue, which is modified/inactivated by modifying the essential lysine residue. Expression of the mutant polypeptide of the invention may then convert the inactivated enzyme to its active form. Conversion then allows the organism, preferably the microorganism, to produce the said essential factor so that all components necessary for growth are present. In a preferred embodiment of the invention, the host cell, preferably the microorganism, lacks a gene encoding pyrF and/or cobB. Such a selection system can be used to identify a KDAC variant, i.e. a mutant polypeptide of the invention, with the ability to revert the modification of the lysine residue in a pool of inactive mutants.

In such an embodiment, the organism according to the invention is an organism, preferably a microorganism, which lacks a gene encoding pyrF and/or cobB and which is recombinant in the sense that it has further been genetically modified so as to express a mutant polypeptide according to the present invention. Thus, the term "recombinant" means that the organism is genetically modified so as to contain a foreign nucleic acid molecule encoding a KDAC variant enzyme of the present invention as defined above. The term "foreign" in this context means that the nucleic acid molecule does not naturally occur in said organism/microorganism. This means that it does not occur in the same structure or at the same location in the organism/microorganism. In one preferred embodiment, the foreign nucleic acid molecule is a recombinant molecule comprising a promoter and a coding sequence encoding the KDAC variant, in which the promoter driving expression of the coding sequence is heterologous with respect to the coding sequence. Heterologous in this context means that the promoter is not the promoter naturally driving the expression of said coding sequence but is a promoter naturally driving expression of a different coding sequence, i.e., it is derived from another gene, or is a synthetic promoter or a chimeric promoter. Preferably, the promoter is a promoter heterologous to the organism/microorganism, i.e. a promoter which does not naturally occur in the respective organism/microorganism. Even more preferably, the promoter is an inducible promoter.

Promoters for driving expression in different types of organisms, in particular in microorganisms, are well known to the person skilled in the art.
In another preferred embodiment the nucleic acid molecule is foreign to the organism/microorganism in that the encoded KDAC variant, is/are not endogenous to the organism/microorganism, i.e. are naturally not expressed by the organism/microorganism when it is not genetically modified.
The term "recombinant" in another embodiment means that the organism is genetically modified in the regulatory region controlling the expression of an enzyme as defined above which naturally occurs in the organism so as to lead to an increase in expression of the respective enzyme in comparison to a corresponding non-genetically modified organism. Such a modification of a regulatory region can be achieved by methods known to the person skilled in the art. One example is to exchange the naturally occurring promoter by a promoter which allows for a higher expression or to modify the naturally occurring promoter so as to show a higher expression. Thus, in this embodiment the organism contains in the regulatory region of the gene encoding an enzyme as defined above a foreign nucleic acid molecule which naturally does not occur in the organism and which leads to a higher expression of the enzyme in comparison to a corresponding non-genetically modified organism.
The foreign nucleic acid molecule may be present in the organism/microorganism in extrachromosomal form, e.g. as plasmid, or stably integrated in the chromosome. A stable integration is preferred.

Methods for preparing the above mentioned genetically modified organism, preferably microorganisms, are well known in the art. Thus, generally, the organism/microorganism is transformed with a DNA construct allowing expression of the respective enzyme in the microorganism. Such a construct normally comprises the coding sequence in question linked to regulatory sequences allowing transcription and translation in the respective host cell, e.g. a promoter and/enhancer and/or transcription terminator and/or ribosome binding sites etc.

The mutant polypeptide of the invention may be used in therapy. In this respect, the mutant polypeptides of the invention may preferably be combined, either in one or separate formulations, with a peptide or polypeptide comprising an inactive essential lysine residue for use in treating cancer.

The term "peptide" generally refers to a contiguous and relatively short sequence of amino acids linked by peptidyl bonds. Typically, but not necessarily, a peptide has a length of about 2 to 50 amino acids, 4-40 amino acids or 10-30 amino acids. Although the term "polypeptide" generally refers to longer forms of a peptide, the two terms can be and are used interchangeably in some contexts herein.
The terms "amino acid" and "residue" are used interchangeably herein. A "region" of a polypeptide is a contiguous sequence of 2 or more amino acids. In other embodiments, a region is at least about any of 3, 5, 10, 15 contiguous amino acids.

Cancer, as used herein, is a group of diseases involving abnormal cell growth with the potential to invade or spread to other parts of the body. Possible signs and symptoms of cancer include a lump, abnormal bleeding, prolonged cough, unexplained weight loss, and a change in bowel movements.
Cancers are often described by the body part that they originated in. However, some body parts contain multiple types of tissue, so for greater precision, cancers are additionally classified by the type of cell that the tumor cells originated from. Types of cancer, which may be treated using the treatment provided by the present invention include: Carcinoma which relates to cancers derived from epithelial cells. This group includes many of the most common cancers, particularly in older adults. Nearly all cancers developing in the breast, prostate, lung, pancreas, and colon are carcinomas; Sarcoma, relating to cancers arising from connective tissue (i.e. bone, cartilage, fat, nerve), each of which develop from cells originating in mesenchymal cells outside the bone marrow; Lymphoma and leukemia, which relate to two classes of cancer that arise from cells that make blood; germ cell tumors which are derived from pluripotent cells, most often presenting in the testicle or the ovary (seminoma and dysgerminoma, respectively); and/or blastoma, i.e. cancers derived from immature "precursor" cells or embryonic tissue.

As used herein, "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, modulatory compounds of the invention are used to delay development of a disease or disorder.
An "individual" or "subject" is a mammal. Mammals include, but are not limited to, domesticated animals (e.g., cows, sheep, cats, dogs, and horses), primates (e.g., humans and non- human primates such as monkeys), rabbits, and rodents (e.g. , mice and rats). In certain embodiments, the individual or subject is a human.

The means of the present invention used for treatment of cancer are preferably applied in an effective amount to treat cancer. In this respect, the term "effective amount" refers to an amount effective, at dosages and for periods of time necessary, to achieve the desired therapeutic or prophylactic result. A "therapeutically effective amount" of a substance/molecule of the invention, agonist or antagonist may vary according to factors such as the disease state, age, sex, and weight of the individual, and the ability of the substance/molecule, agonist or antagonist to elicit a desired response in the individual. A therapeutically effective amount is also one in which any toxic or detrimental effects of the substance/molecule, agonist or antagonist are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.
The mutant polypeptide of the invention and/or the peptide/polypeptide comprising an inactive essential lysine residue may be part of a pharmaceutical formulation. The term "pharmaceutical formulation" refers to a preparation which is in such form as to permit the biological activity of an active ingredient contained therein to be effective, and which contains no additional components which are unacceptably toxic to a subject to which the formulation would be administered.
Such formulations may comprise a pharmaceutically acceptable carrier. A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.
The term "package insert" is used to refer to instructions customarily included in commercial packages of therapeutic products, that contain information about the indications, usage, dosage, administration, combination therapy, contraindications and/or warnings concerning the use of such therapeutic products.

The mutant polypeptides of the invention may be in isolated and/or purified form. An "isolated" or "purified" peptide, polypeptide, protein or biologically active fragment is separated and/or recovered from a component of its natural environment. Contaminant components include materials that would typically interfere with diagnostic or therapeutic uses for the polypeptide, and may include enzymes, hormones, and other proteinaceous or non-proteinaceous materials. Preparations having preferably less than 30% by dry weight of non- desired contaminating material (contaminants), preferably less than 20%, 10%, and preferably less than 5% contaminants are considered to be substantially isolated. An isolated, recombinantly-produced peptide/polypeptide or biologically active portion thereof is preferably substantially free of culture medium, i.e., culture medium represents preferably less than 20%, preferably less than about 10%, and preferably less than about 5% of the volume of a peptide/polypeptide preparation. Examples of contaminants include cell debris, culture media, and substances used and produced during in vitro synthesis of the peptide/polypeptide.

A mutant polypeptide and/or the peptide/polypeptide comprising an inactive essential lysine residue of the invention can be incorporated into compositions, which in some embodiments are suitable for pharmaceutical use. Such compositions typically comprise the peptides and/or polypeptides, and an acceptable carrier, for example one that is pharmaceutically acceptable. A "pharmaceutically acceptable carrier" includes any and all solvents, dispersion media, coatings, antibacterial and antifungal agents, isotonic and absorption delaying agents, and the like, compatible with pharmaceutical administration (Gennaro, Remington: The science and practice of pharmacy. Lippincott, Williams & Wilkins, Philadelphia, Pa. (2000)). Examples of such carriers or diluents include, but are not limited to, water, saline, Finger's solutions, dextrose solution, and 5% human serum albumin. Liposomes and non-aqueous vehicles such as fixed oils may also be used. Except when a conventional media or agent is incompatible with an active compound, use of these compositions is contemplated. Supplementary active compounds can also be incorporated into the compositions.

A pharmaceutical composition is formulated to be compatible with its intended route of administration, including intravenous, intradermal, subcutaneous, oral (e.g., inhalation), transdermal (i.e., topical), transmucosal, and rectal administration. Solutions or suspensions used for parenteral, intradermal, or subcutaneous application can include: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampules, disposable syringes or multiple dose vials made of glass or plastic.

Pharmaceutical compositions suitable for injection include sterile aqueous solutions (where water soluble) or dispersions and sterile powders for the extemporaneous preparation of sterile injectable solutions or dispersion. For intravenous administration, suitable carriers include physiological saline, bacteriostatic water, CREMOPHOR EL^{1 M} (BASF, Parsippany, N.J.) or phosphate buffered saline (PBS). In all cases, the composition must be sterile and should be fluid so as to be administered using a syringe. Such compositions should be stable during manufacture and storage and must be preserved against contamination from microorganisms such as bacteria and fungi. The carrier can be a solvent or dispersion medium containing, for example, water, ethanol, polyol (such as glycerol, propylene glycol, and liquid polyethylene glycol), and suitable mixtures. Proper fluidity can be maintained, for example, by using a coating such as lecithin, by maintaining the required particle size in the case of dispersion and by using surfactants. Various antibacterial and antifungal agents; for example, parabens, chlorobutanol, phenol, ascorbic acid, and thimerosal, can contain microorganism contamination. Isotonic agents; for example, sugars, polyalcohols such as manitol, sorbitol, and sodium chloride can be included in the composition. Compositions that can delay absorption include agents such as aluminum monostearate and gelatin.

Sterile injectable solutions can be prepared by incorporating the active compound (e.g., any modulator substance/molecule of the invention) in the required amount in an appropriate solvent with one or a combination of ingredients as required, followed by sterilization.

Generally, dispersions are prepared by incorporating the active compound into a sterile vehicle that contains a basic dispersion medium, and the other required ingredients. Sterile powders for the preparation of sterile injectable solutions, methods of preparation include vacuum drying and freeze-drying that yield a powder containing the active ingredient and any desired ingredient from a sterile solutions.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally. Pharmaceutically compatible binding agents, and/or adjuvant materials can be included. Tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, PRIMOGEL, or corn starch; a lubricant such as magnesium stearate or STEROTES; a glidant such as colloidal silicon dioxide; a sweetening agent such as sucrose or saccharin; or a flavoring agent such as peppermint, methyl salicylate, or orange flavoring.

For administration by inhalation, the compounds are delivered as an aerosol spray from a nebulizer or a pressurized container that contains a suitable propellant, e.g., a gas such as carbon dioxide.

Systemic administration can also be transmucosal or transdermal. For transmucosal or transdermal administration, penetrants that can permeate the target barrier(s) are selected.

Transmucosal penetrants include, detergents, bile salts, and fusidic acid derivatives. Nasal sprays or suppositories can be used for transmucosal administration. For transdermal administration, the active compounds are formulated into ointments, salves, gels, or creams.

The compounds can also be prepared in the form of suppositories (e.g., with bases such as cocoa butter and other glycerides) or retention enemas for rectal delivery.

In one embodiment, the active compounds are prepared with carriers that protect the compound against rapid elimination from the body, such as a controlled release formulation, including implants and microencapsulated delivery systems. Biodegradable or biocompatible polymers can be used, such as ethylene vinyl acetate, polyanhydrides, polyglycolic acid, collagen, polyorthoesters, and polylactic acid. Such materials can be obtained commercially from ALZA Corporation (Mountain View, Calif.) and NOVA Pharmaceuticals, Inc. (Lake Elsinore, Calif), or prepared by one of skill in the art. Liposomal suspensions can also be used as pharmaceutically acceptable carriers. These can be prepared according to methods known to those skilled in the art, such as in (Eppstein et al , U.S. Pat. No. 4,522,81 1, 1985). 7. Unit Dosage

Oral formulations or parenteral compositions in unit dosage form can be created to facilitate administration and dosage uniformity. Unit dosage form refers to physically discrete units suited as single dosages for the subject to be treated, containing a therapeutically effective quantity of active compound in association with the required pharmaceutical carrier. The specification for the unit dosage forms are dictated by, and directly dependent on, the unique characteristics of the active compound and the particular desired therapeutic effect, and the inherent limitations of compounding the active compound.

The polypeptides/peptides/compositions of the invention may be administered together or sequentially.

Nucleic acid molecules encoding peptides or polypeptides of the invention can be inserted into vectors and used as gene therapy vectors. Gene therapy vectors can be delivered to a subject by, for example, intravenous injection, local administration (Nabel and Nabel, U.S. Pat. No. 5,328,470, 1994), or by stereotactic injection (Chen et al , Proc Natl Acad Sci USA. 91 ; 3054-7 (1994)). The pharmaceutical preparation of a gene therapy vector can include an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells that produce the gene delivery system.

Any of the mutant polypeptides provided herein may be used in therapeutic methods.

In one aspect, a mutant KDAC polypeptide and a peptide/polypeptide comprising an inactive essential lysine residue for use as a medicament is provided. In one such embodiment, the method further comprises administering to the individual an effective amount of at least one additional therapeutic agent. An "individual" according to any of the above embodiments is preferably a human.

In a further aspect, the invention provides for the use of a mutant polypeptide of the invention in the manufacture or preparation of a medicament. In one embodiment, the medicament is for treatment of cancer. In a further embodiment, the medicament is for use in a method of treating cancer comprising administering to an individual having cancer an effective amount of the medicament. In one such embodiment, the method further comprises administering to the individual an effective amount of at least one additional therapeutic agent, e.g. , as described below.

In a further aspect, the invention provides pharmaceutical formulations comprising any of the mutant polypeptides and/or the peptide/polypeptide provided herein, e.g. for use in any of the above therapeutic methods. In one embodiment, a pharmaceutical formulation comprises a pharmaceutically acceptable carrier. In another embodiment, a pharmaceutical formulation comprises at least one additional therapeutic agent, e.g. , as described below.

Peptides or polypeptides of the invention can be used either alone or in combination with other agents in a therapy. For instance, a peptide or polypeptide of the invention may be coadministered with at least one additional therapeutic agent.

Such combination therapies noted above encompass combined administration (where two or more therapeutic agents are included in the same or separate formulations), and separate administration, in which case, administration of the polypeptide of the invention can occur prior to, simultaneously, and/or following, administration of the additional therapeutic agent and/or adjuvant. Peptides or polypeptides of the invention can also be used in combination with radiation therapy.

A peptide or polypeptide of the invention (and any additional therapeutic agent) can be administered by any suitable means, including parenteral, intrapulmonary, intrathecal and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time- points, bolus administration, and pulse infusion are contemplated herein.

Certain embodiments of the invention provide for the peptide or polypeptide to traverse the blood-brain barrier. Certain cancer diseases are associated with an increase in permeability of the blood-brain barrier, such that the peptide or polypeptide can be readily introduced to the brain. When the blood-brain barrier remains intact, several art-known approaches exist for transporting molecules across it, including, but not limited to, physical methods, lipid-based methods, and receptor and channel-based methods.

Physical methods of transporting the peptide or polypeptide across the blood-brain barrier include, but are not limited to, circumventing the blood-brain barrier entirely, or by creating openings in the blood-brain barrier. Circumvention methods include, but are not limited to, direct injection into the brain (see e.g. , Papanastassiou et al., Gene Therapy 9: 398-406 (2002)) and implanting a delivery device in the brain (see e.g., Gill et al., Nature Med. 9: 589- 556 (2003); and Gliadel Wafers™, Guildford Pharmaceutical). Methods of creating openings in the barrier include, but are not limited to, ultrasound (see e.g. , U.S. Patent Publication No. 2002/0038086), osmotic pressure (e.g. , by administration of hypertonic mannitol (Neuwelt, E. A., Implication of the Blood-Brain Barrier and its Manipulation, Vols 1 & 2, Plenum Press, N.Y. (1989))), permeabilization by, e.g. , bradykinin or permeabilizer A-7 (see e.g. , U.S. Pat. Nos. 5,1 12,596, 5,268,164, 5,506,206, and 5,686,416), and transfection of neurons that straddle the blood-brain barrier with vectors containing genes encoding the antibody or fragment thereof (see e.g. , U.S. Patent Publication No. 2003/0083299).

Peptides or polypeptides of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The peptide or polypeptide need not be, but is optionally formulated with one or more agents currently used to prevent or treat the disorder in question, in particular cancer. The effective amount of such other agents depends on the amount of polypeptide present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, in particular cancer, the appropriate dosage of a peptide or polypeptide of the invention (when used alone or in combination with one or more other additional therapeutic agents) will depend on the type of cancer to be treated, the severity and course of the cancer, whether the peptide or polypeptide is administered for preventive or therapeutic purposes, previous therapy, the patient's clinical history and response to the peptide or polypeptide, and the discretion of the attending physician. The peptide or polypeptide is suitably administered to the patient at one time or over a series of treatments. Depending on the type and severity of the disease, an appropriate dosage level will generally be about 0.01 to 500 mg per kg patient body weight per day which can be administered in single or multiple doses. Preferably, the dosage level will be about 0.1 to about 250 mg/kg per day; more preferably about 0.5 to about 100 mg/kg per day. A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5 or 5 to 50 mg/kg per day. For oral administration, the compositions are preferably provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, and 1000.0 milligrams of the active ingredient for the symptomatic adjustment of the dosage to the patient to be treated. The compounds may be administered on a regimen of 1 to 4 times per day, preferably once or twice per day.

However, the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy. The progress of this therapy is easily monitored by conventional techniques and assays.

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a peptide or polypeptide of the invention. The label or package insert indicates that the composition is used for treating the condition of choice. Moreover, the article of manufacture may comprise (a) a first container with a composition contained therein, wherein the composition comprises a peptide or polypeptide of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes. The following examples are included to demonstrate preferred embodiments of the present invention. It should be appreciated by those of skill in the art that the techniques disclosed in the examples that follow represent techniques discovered by the inventors to function well in the practice of the invention, and thus can be considered to constitute preferred modes for its practice. However, those of skill in the art should, in light of the present disclosure, appreciate that many changes can be made in the specific embodiments that are disclosed and still obtain a like or similar result without departing form the spirit and scope of the invention.

In one embodiment, the inactivated lysine residue of the peptide or polypeptide of the invention comprising an essential lysine residue is acetylated or comprises a protection group.

Within the present invention, the term "acetylation" describes a reaction that introduces an acetyl functional group into a chemical compound. "Deacetylation" is the removal of an acetyl group.

Acetylation refers to the process of introducing an acetyl group (resulting in an acetoxy group) into a compound, namely the substitution of an acetyl group for an active hydrogen atom. A reaction involving the replacement of the hydrogen atom of a hydroxyl group with an acetyl group (CH₃ CO) yields a specific ester, the acetate. Acetic anhydride is commonly used as an acetylating agent reacting with free hydroxyl groups. For example, it is used in the synthesis of aspirin, heroin, and THC-O-acetate.

Proteins are typically acetylated on lysine residues and this reaction relies, in vivo, on acetyl-coenzyme A. However, proteins can also artificially be acetylated. In histone acetylation and deacetylation, histone proteins are acetylated and deacetylated on lysine residues in the N-terminal tail as part of gene regulation. The regulation of transcription factors, effector proteins, molecular chaperones, and cytoskeletal proteins by acetylation and deacetylation is a significant post-translational regulatory mechanism. These regulatory mechanisms are analogous to phosphorylation and dephosphorylation by the action of kinases and phosphatases. Not only can the acetylation state of a protein modify its activity but there has been recent suggestion that this post-translational modification may also crosstalk with phosphorylation, methylation, ubiquitination, sumoylation, and others for dynamic control of cellular signaling.

If an essential lysine residue, i.e. a lysine residue required for the natural activity of the acetylated polypeptide, is acetylated, it will in some cases loose its activity or show a reduced activity. Therefore, the peptide or polypeptide comprising an essential lysine residue of the invention is named "inactive" due to the acetylation. In this respect, "inactive" means that the peptide or polypeptide does not show its natural activity to the same extent as in its "active" form, i.e. without being acetylated at the essential lysine residue. The activity may be reduced due to acetylation from 100% to 90%, 80%, 70%, 60%, 50%, 40%, 30%, 20%, 10% or even 0%. It is preferred that the activity is reduced to a minimum.

The essential lysine residue of the peptide or polypeptide comprising an essential lysine residue of the invention may also be inactivated by alternative protection groups. Such protection groups are generally known in the art and every protection group is possible as long as it can be removed by the mutant polypeptide having lysine demodification activity of the invention. Such protection groups may be N(ε)-tert.-butyloxycarbonyl (Boc), N(ε)-allyloxycarbonyl (Aloc), N(ε)-propargyloxycarbonyl (Poc), N(ε)-benzyloxycarbonyl (Z), N(s)-2,2,2-trichloroethyloxycarbonyl (Troc), N(ε)-azidomethoxycarbonyl (Azoc), N(ε)-2-chlorobenzyloxycarbonyl (CI-Z) or N(ε)-trifluoroacetyl (tfa).

The invention furthermore relates to a method of screening for a mutant polypeptide having lysine demodification activity, wherein the method comprises the following steps (a) incubating a mutant polypeptide having an amino acid sequence with at least 80% sequence identity to SEQ ID NO: 1 with a peptide or polypeptide comprising an inactivated essential lysine residue; and (b) determining the activity of the mutant polypeptide to activate the peptide or polypeptide comprising the inactivated essential lysine residue, wherein the mutant polypeptide and the peptide or polypeptide comprising an inactivated essential lysine residue are incubated in a biological cell.

Accordingly, a selection system for KDACs with altered substrate specificity and/or reactivity against bioorthogonal chemical protection groups is reported. The system builds on the incorporation of lysine derivatives by genetic code expansion in reporter enzymes with essential active site lysine residues. The reporter enzyme containing the lysine derivative is an inactive precursor that is turned on upon removal of the modification, thereby coupling deacetylase activity to a selectable output. This enables to evolve KDACs selective for particular lysine acylations and other bioorthogonal modifications. These KDAC variants may be used to partially complement KDAC deletion strains or to design a prodrug strategy for cancer therapy.

The invention is based on a selection system for lysine deacetylases (KDACs) based on a selectable marker that contains an essential lysine residue. By replacing this residue with modified forms of lysine (e.g. acetylated or with protection groups) using genetic code expansion, we generate an inactive precursor enzyme. Cells must revert the modification to activate the selectable marker, hence coupling KDAC activity to cell survival. Using this system, KDAC variants with increased substrate specificity or the ability to remove protection groups from lysine residues could be created and are provided herein.

Here, the directed evolution of KDACs towards particular acyl substrates and bioorthogonal lysine modifications using a bacterial selection system is reported. The new polypeptides of the invention can be used for partial complementation of KDAC deletion strains to reveal the physiological role of particular lysine acylations. Bioorthogonal "eraser" enzymes facilitate the activation of pro-peptides or pro-enzymes by removing protection groups installed on lysine residues. These bioorthogonal "eraser" enzymes may therefore find applications in prodrug strategies of cancer therapy.

The screening method of the invention may be carried out in any biological cell, preferably a bacterial cell. Accordingly, in one embodiment, the invention relates to a method of screening for a mutant polypeptide having lysine demodification activity, wherein the method comprises the following steps (a) incubating a mutant polypeptide having an amino acid sequence with at least 80% sequence identity to SEQ ID NO: 1 with a peptide or polypeptide comprising an inactivated essential lysine residue; and (b) determining the activity of the mutant polypeptide to activate the peptide or polypeptide comprising the inactivated essential lysine residue, wherein the mutant polypeptide and the peptide or polypeptide comprising an inactivated essential lysine residue are incubated in a bacterial cell. However, the screening method of the invention is not limited to sequences having 80% identity to SEQ ID NO:1. That is, the starting sequence does not have to be related to CobB, which is an example of sirtuines. The method of the invention can also be based on alternative sequences, for example, starting from HDAC8 or other zinc dependent enzymes.

The bacterial cell is preferably E. coli. In order to determine the activity of the mutant polypeptide having lysine demodification activity, it is preferred that the E. coli cell lacks a gene encoding for pyrF and/or cobB. This is because lysine demodification activity of the mutant polypeptide to be screened can then surprisingly and unexpectedly well correlated with the activity of the mutant polypeptide to be screened. In a preferred embodiment, the mutant polypeptide is not identical to SEQ ID NO:1.

In order to provide a screening method, which can surprisingly and unexpectedly well determine the lysine demodification activity of a mutant polypeptide to be screened, a reporter gene is used, which leads to a detectable and quantifiable signal. In this respect, the skilled person can select reporter genes as long as said reporter gene carries an essential lysine residue, which can be modified and subsequently demodified by the mutant polypeptide of interest. It is preferred that the peptide or polypeptide comprising an inactivated essential lysine residue is OMP decarboxylase or Firefly luciferase. In this respect, it is preferred that OMP decarboxylase is buddying yeast OMP decarboxylase (Ura3) or E. coli pyrF. The essential lysine residue carried by the reporter gene can be inactivated by acetylation or a protection group, as described further above.

The invention furthermore relates to devices for carrying out the screening method, in particular devices used for high-throughput screening.

The invention also relates to an E. coli strain lacking expression of pyrF and cobB. Preferably, the the E. coli strain of the invention expresses Ura3 comprising a modified essential lysine residue.

The invention also relates to a kit comprising the E. coli strain of the invention and/or the mutant polypeptide of the invention.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

The general methods and techniques described herein may be performed according to conventional methods well known in the art and as described in various general and more specific references that are cited and discussed throughout the present specification unless otherwise indicated. See, e.g., Sambrook et al., Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1563), and Harlow and Lane Antibodies: A Laboratory Manual, Cold Spring Harbor Laboratory Press, Cold Spring Harbor, N.Y. (1990).

While aspects of the invention are illustrated and described in detail in the drawings and foregoing description, such illustration and description are to be considered illustrative or exemplary and not restrictive. It will be understood that changes and modifications may be made by those of ordinary skill within the scope and spirit of the following claims. In particular, the present invention covers further embodiments with any combination of features from different embodiments described above and below. The invention also covers all further features shown in the figures individually, although they may not have been described in the previous or following description. Also, single alternatives of the embodiments described in the figures and the description and single alternatives of features thereof can be disclaimed from the subject matter of the other aspect of the invention.

Furthermore, in the claims the word "comprising" does not exclude other elements or steps, and the indefinite article "a" or "an" does not exclude a plurality. A single unit may fulfill the functions of several features recited in the claims. The terms "essentially", "about", "approximately" and the like in connection with an attribute or a value particularly also define exactly the attribute or exactly the value, respectively. Any reference signs in the claims should not be construed as limiting the scope.

Aspects of the present invention are additionally described by way of the following illustrative non-limiting examples that provide a better understanding of embodiments of the present invention and of its many advantages. The following examples are included to demonstrate preferred embodiments of the invention. It should be appreciated by those of skill in the art that the techniques disclosed in the examples which follow represent techniques used in the present invention to function well in the practice of the invention, and thus can be considered to constitute preferred modes for its practice. However, those of skill in the art should appreciate, in light of the present disclosure that many changes can be made in the specific embodiments which are disclosed and still obtain a like or similar result without departing from the spirit and scope of the invention. A number of documents including patent applications, manufacturer's manuals and scientific publications are cited herein. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

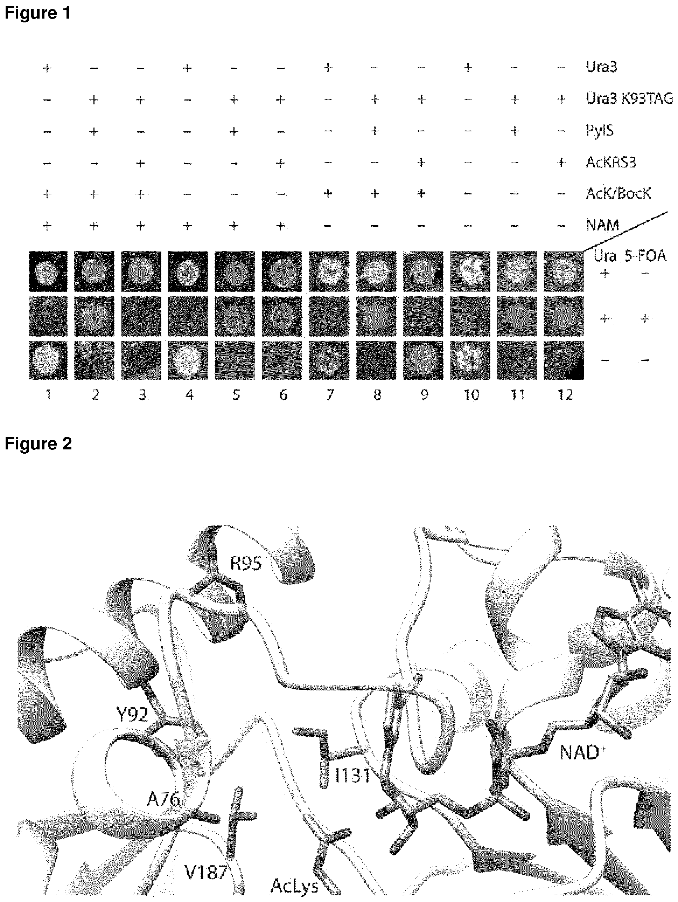

**Figure 1****:** *E. coli* producing Ura3 K93ac as the sole source of OMP decarboxylase depend on KDAC activity. *E. coli* DB6566 (ΔpyrF) expressing plasmids to encode wild-type Ura3, ra3 K93ac or K93boc were plated on agar plates with or without uracil, 5-FOA and the corresponding unnatural amino acid. Nicotinamide (NAM) was added to inhibit endogenous CobB.
**Figure 2****:** CobB library design. Highlighted amino acid residues were randomized to all possible combinations of natural amino acids to generate a library of thirty million mutants in the active site of CobB.
**Figure 3****:** Evolved CobB variants can activate Firefly luciferase modified at K529. Dual luciferase reporter (DLR) assays were performed with *E. coli* producing DLR with the indicated modification of K529 in the Firefly enzyme. Activities were normalized to genetically fused Renilla luciferase and plotted relative to the activity observed for wild-type CobB on the same substrate. A) CobB mutant able to discriminate crotonyl- over acetyl-lysine. B) CobB mutants active against protected forms of lysine.

### Example 1 - Design of a selection system for lysine deacetylases

To develop a selection system for KDACs enzymes with lysine residues essential for activity were searched. Of the enzymes two were tested, orotidine-5'-phosphate (OMP) decarboxylase and firefly luciferase. Both proved to be suitable as selectable marker and reporter enzymes, respectively. When N(ε)-acetyl-lysine was incorporated in place of K93 of budding yeast OMP decarboxylase (Ura3), the protein was unable to support growth of *E. coli cells* lacking pyrF (the homologue of Ura3) and cobB (the major lysine deacetylase of *E*. *coli,* inhibited with nicotinamide) in the absence of uracil (Figure 1, column 3, bottom). In the presence of cobB robust growth was observed on minimal medium without uracil, indicating that cobB was able to remove the acetyl group from the active site lysine of Ura3 K93ac. Growth of the same cells is inhibited when 5-fluoro-orotic acid, a compound converted into a toxic metabolite by Ura3, is added to the medium. Hence, this system is able to positively and negatively select *E*. *coli* harbouring an active lysine deacetylase. The same system can also be used to select for HDAC8 activity, a mammalian class I lysine deacetylase structurally and mechanistically distinct from the sirtuin family member cobB. Firefly luciferase contains an essential lysine residue (K529) in the active site. Replacing this residue by genetic code expansion with N(ε)-acetyl-lysine rendered the enzyme inactive in the absence of lysine deacetylase cobB. In the presence of cobB robust activity of the enzyme was observed. Hence, K529ac firefly luciferase can be used to screen for lysine deacetylase activity in *E. coli.*

### Example 2 - Creation of cobB mutant libraries

Next, a mutant library was created by randomizing five active site residues (A37, Y53, R56, 192 and V148) of *E*. *coli* cobB to all possible combinations of natural amino acids, thereby creating 20⁵ (3.2x10⁶) different mutants (Figure 2).

### Example 3 - Isolation of acyl-type specific deacetylases

To identify cobB mutants selectively removing crotonyl but not acetyl groups, the library was subjected to two rounds of selection, positive and negative. Therefore, *E. coli* DH10B ΔpyrF ΔcobB harbouring a reporter plasmid encoding ura3 K93TAG together with wildtype *Mb*PylRS and the cognate amber suppressor tRNA *Mb*PylT was transformed with the cobB mutant library. The cells were challenged to grow in the presence of N(ε)-crotonyl-lysine on medium without uracil to select clones able to decrotonylate Ura3 K93ac. Library plasmids were isolated from the pool of surviving clones and used to transform DH10B ΔpyrF ΔcobB harbouring a reporter plasmid encoding AcKRS3 (*M. barkeri* PylRS variant encoding N(ε)-acetyl-lysine) instead of *Mb*PylRS. Cells were grown on plates containing N(ε)-acetyl-lysine and 5-fluoro-orotic acid (5-FOA), which is toxic to cells in the presence of active Ura3, to select against clones able to remove acetyl groups from Ura3 K93cr. Individual clones were arrayed and tested for the ability to survive on medium without uracil in the presence of N(ε)-crotonyl-lysine. Thereby several mutants of CobB were identified that were able to selectively cleave crotonyl but not acetyl groups off lysine side chains.

### Example 4 - Selection of bioorthogonal Eraser enzymes

Next, the same cobB mutant library was challenged to remove chemical protection groups from lysine residues. N(ε)-tert.-butyl-oxycarbonyl-lysine (BocK), N(ε)-allyl-oxycarbonyl-lysine (AlocK) and N(ε)-propargyl-oxycarbonyl-lysine (PrK) can be incorporated in proteins using wild-type PylRS/PylT. *E. coli* DH10B ΔpyrF ΔcobB harbouring the mutant library was challenged to grow in the absence of uracil while incorporating one of these unnatural amino acids in Ura3 in place of K93. Surviving clones were arrayed and plasmids isolated from cells that grew in the absence of uracil depending on the presence of one of the unnatural amino acids. Several mutants capable of cleaving AlocK were identified and a single mutant with activity against BocK (Table 1). Individual testing of mutants isolated in the BocK and AlocK selections for activity against PrK revealed several mutants with basal activity.

### Example 5 - Quantitative analysis of mutant activities using Firefly luciferase assay

The mutants isolated in the selections were tested using Firefly dual luciferase assays. *E.coli* DH10B ΔpyrF ΔcobB were transformed with plasmids expressing Firefly luciferase with the relevant modification on lysine-529 and the cobB mutants. Luciferase activity was tested directly in whole cell lysates and compared to the activity of wild-type cobB towards the modifications. The activities observed for the evolved KDAC variants correlated well with the activities observed in the uracil selections (Figure 3).

The selection system of the invention is capable of identifying an individual KDAC variant with the desired activity in a library of more than three million mutants in a single round. Enzymes could be identified to remove typical protection groups for lysine side chains active enough to activate a sufficient amount of Ura3 enzyme to sustain cell growth in the absence of uracil. The selection system can be easily modified to select other KDAC mutant libraries and other lysine modifications. It may also be used to design selective mutant/inhibitor pairs by a bump-and-hole strategy. Enzymes catalysing bioorthogonal reactions are the key to success for the development of save prodrug strategies in cancer therapy. Presently, enzymes to activate prodrugs are either of human origin (with the disadvantage of being present in other tissues and therefore causing side effects) or from a different organism (with the disadvantage of being immunogenic). KDAC variants of the invention with bioorthogonal activity evolved from a parent enzyme of human origin combine the advantages of both approaches.

**Table 1**

| | | Growth on -ura | | | | | DLR activity [rel. to wild-type] | | | |
|---|---|---|---|---|---|---|---|---|---|---|
| **Name** | **Mutations** | **AcK** | **CrK** | **BocK** | **AlocK** | **PrK** | **AcK** | **CrK** | **BocK** | **AlocK** |
| Dealocase-1 SEQ ID 4 | A37S Y53W R56W I92V V148V | n.d. | + | + | + | - | 0,49 | 5,73 | 2,88 | 10,83 |
| Dealocase-2 SEQ ID 5 | A37G R56G I92V | n.d. | + | + | - | - | 1,19 | 2,11 | 0,77 | 10,23 |
| Dealocase-3 SEQ ID 6 | I1315 V148L | - | + | - | + | n.d. | 0,35 | 1,00 | 0,65 | 6,00 |
| Debocylase-1 SEQ ID 7 | I131V V148L | + | + | + | + | + | 1,25 | 2,90 | 8,65 | 8,35 |
| Decrotonylase-1 SEQ ID 8 | A37R Y53G R56T I92R V148L | - | + | - | + | n.d. | 0,00 | 0,10 | 0,04 | 0,02 |

### Example 6 - Development of humanized variants

Humanized deacetlyases, i.e. mutant polypeptide of the invention, have been developed. The advantage of a human origin is that there will be no, or only a very reduced, immune reaction in the human organism. For this purpose, the enzymes SirT1, SirT2 and SirT3 are cloned in an analogous manner to E. coli cobB. Cloned enzymes are characterized for their ability to activate the marker protein Ura3 K93ac by demodifying the essential lysine residue. Subsequently, mutant libraries are built based on the active variant enzymes. This process is identical to the above-described process based on E. coli cobB.

Inactive precursor molecules of toxic substances are used in cancer therapy, as it is part of the present invention. For this purpose, toxic peptides are modified at their essential lysine residues using protection groups, acetylation and the like. The resulting peptides are tested on human cell lines for toxicity, whereby a low toxicity is preferred. The evolved deacetylases are then characterized for their ability to remove the protection groups and to activate the pro-toxin.

The evolved human deacetylases are tested in human cancer cell lines. For this purpose, the polypeptides are expressed in those cell lines. Subsequently, the cell lines are administered with the pro-toxin peptides to test the ability of the deacetylases to activate them and to provide its effects on the cancer cell line.

## Claims

1. A mutant polypeptide comprising an amino acid sequence having at least 98% sequence homology with SEQ ID NOs: 4, 5, 6, 7 or 8 and having lysine demodification activity, wherein the mutant polypeptide is not identical to SEQ ID NO: 1.

2. The mutant polypeptide of claim 1 or 2, wherein the mutant polypeptide comprises an amino acid sequence having 1 to 5 mutations in the amino acid sequence of SEQ ID NO: 1.

3. The mutant polypeptide of claim 1 or 2, wherein the mutant polypeptide comprises one or more mutation(s) at positions 37, 53, 56, 92 and/or 148 of SEQ ID NO:1.

4. The mutant polypeptide of claim 3, wherein the polypeptide comprises mutations A37S, Y53W, R56W, I92V and/or V148L with respect to SEQ ID NO: 1.

5. The mutant polypeptide of any one of claims 1 to 4, which is SEQ ID NO: 4, 5, 6, 7 or 8.

6. The mutant polypeptide of claim 1 and a peptide or polypeptide comprising an inactivated essential lysine residue for use in treating cancer.

7. The mutant polypeptide and the peptide or polypeptide for use of claim 6, wherein the mutant polypeptide and the peptide or polypeptide are administered together or sequentially.

8. The mutant polypeptide and the peptide or polypeptide for use of claim 6 or 7, wherein the inactivated lysine residue of the peptide or polypeptide is acetylated or comprises a protection group.

9. A method of screening for a mutant polypeptide having lysine demodification activity, wherein the method comprises the following steps:
(a) incubating a mutant polypeptide having an amino acid sequence with at least 80% sequence identity to SEQ ID NO: 1 with a peptide or polypeptide comprising an inactivated essential lysine residue; and
(b) determining the activity of the mutant polypeptide to activate the peptide or polypeptide comprising the inactivated essential lysine residue,
wherein the mutant polypeptide and the peptide or polypeptide comprising an inactivated essential lysine residue are incubated in a biological cell.

10. The method of claim 9, wherein the biological cell is a bacterial cell.

11. The method of claim 10, wherein the bacterial cell is E. coli.

12. The method of claim 10 or 11, wherein the bacterial cells, preferably E. coli cells, lack a gene encoding pyrF and/or cobB.

13. The method of any one of claims 9 to 12, wherein the mutant polypeptide has an amino acid sequence that is not identical to SEQ ID NO:1.

14. The method of any one of claims 9 to 13, wherein the peptide or polypeptide comprising an inactivated essential lysine residue is OMP decarboxylase or Firefly luciferase.

15. The method of claim 14, wherein OMP decarboxylase is buddying yeast OMP decarboxylase (Ura3) or E. coli pyrF.

16. The method of any one of claims 9 to 15, wherein the essential lysine residue is inactivated by acetylation or a protection group.

17. An E. coli strain lacking expression of pyrF and cobB.

18. The E. coli strain of claim 17 expressing Ura3 comprising a modified essential lysine residue.


SEQUENCE LISTING

<110> Max-Planck-Gesellschaft zur Foerderung der Wissenschaften e.V.

<120> KDAC variants and uses therof

<130> AA2372 EP S3

<160> 8

<170> BiSSAP 1.3

<210> 1
<211> 240
<212> PRT
<213> E. coli CobB

<400> 1
Lys Pro Arg Val Leu Val Leu Thr Gly Ala Gly Ile Ser Ala Glu Ser
1               5                   10                  15
Gly Ile Arg Thr Phe Arg Ala Ala Asp Gly Leu Trp Glu Glu His Arg
            20                  25                  30
Val Glu Asp Val Ala Thr Pro Glu Gly Phe Asp Arg Asp Pro Glu Leu
        35                  40                  45
Val Gln Ala Phe Tyr Asn Ala Arg Arg Arg Gln Leu Gln Gln Pro Glu
    50                  55                  60
Ile Gln Pro Asn Ala Ala His Leu Ala Leu Ala Lys Leu Gln Asp Ala
65                  70                  75                  80
Leu Gly Asp Arg Phe Leu Leu Val Thr Gln Asn Ile Asp Asn Leu His
                85                  90                  95
Glu Arg Ala Gly Asn Thr Asn Val Ile His Met His Gly Glu Leu Leu
            100                 105                 110
Lys Val Arg Cys Ser Gln Ser Gly Gln Val Leu Asp Trp Thr Gly Asp
        115                 120                 125
Val Thr Pro Glu Asp Lys Cys His Cys Cys Gln Phe Pro Ala Pro Leu
    130                 135                 140
Arg Pro His Val Val Trp Phe Gly Glu Met Pro Leu Gly Met Asp Glu
145                 150                 155                 160
Ile Tyr Met Ala Leu Ser Met Ala Asp Ile Phe Ile Ala Ile Gly Thr
                165                 170                 175
Ser Gly His Val Tyr Pro Ala Ala Gly Phe Val His Glu Ala Lys Leu
            180                 185                 190
His Gly Ala His Thr Val Glu Leu Asn Leu Glu Pro Ser Gln Val Gly
        195                 200                 205
Asn Glu Phe Ala Glu Lys Tyr Tyr Gly Pro Ala Ser Gln Val Val Pro
    210                 215                 220
Glu Phe Val Glu Lys Leu Leu Lys Gly Leu Lys Ala Gly Ser Ile Ala
225                 230                 235                 240

<210> 2
<211> 765
<212> DNA
<213> Artificial Sequence

<220>
<223> E. coli CobB with His-Tag

<400> 2
atgggcagca gccatcacca tcatcaccac agccaggatc caaaaccaag agtactcgta      60

ctgacagggg caggaatttc tgcggaatca ggtattcgta cctttcgcgc cgcagatggc     120

ctgtgggaag aacatcgggt tgaagatgtg gcaacgccgg aaggtttcga tcgcgatcct 180

gaactggtgc aagcgtttta taacgcccgt cgtcgacagc tgcagcagcc agaaattcag 240

cctaacgccg cgcatcttgc gctggctaaa ctgcaagacg ccctcggcga tcgctttttg 300

ctggtgacgc agaatatcga caacctgcat gaacgcgcag gtaataccaa tgtgattcat 360

atgcatgggg aactgctgaa agtgcgttgt tcacaaagtg gtcaggttct cgactggaca 420

ggagacgtta ccccagaaga taaatgccat tgttgccagt ttccggcacc cttgcgcccg 480

cacgtagtgt ggtttggcga aatgccactc ggcatggatg aaatttatat ggcgttgtcg 540

atggccgata ttttcattgc cattggcaca tccgggcatg tttatccggc ggctgggttt 600

gttcacgaag cgaaactgca tggcgcgcac accgtggaac tgaatcttga accgagtcag 660

gttggtaatg aatttgccga gaaatattac ggcccggcaa gccaggtggt gcctgagttt 720

gttgaaaagt tgctgaaggg attaaaagcg ggaagcattg cctaa 765

<210> 3
<211> 254
<212> PRT
<213> Artificial Sequence

<220>
<223> E. coli CobB with His-Tag

<400> 3
Met Gly Ser Ser His His His His His His Ser Gln Asp Pro Lys Pro
1               5                   10                  15
Arg Val Leu Val Leu Thr Gly Ala Gly Ile Ser Ala Glu Ser Gly Ile
            20                  25                  30
Arg Thr Phe Arg Ala Ala Asp Gly Leu Trp Glu Glu His Arg Val Glu
        35                  40                  45
Asp Val Ala Thr Pro Glu Gly Phe Asp Arg Asp Pro Glu Leu Val Gln
    50                  55                  60
Ala Phe Tyr Asn Ala Arg Arg Arg Gln Leu Gln Gln Pro Glu Ile Gln
65                  70                  75                  80
Pro Asn Ala Ala His Leu Ala Leu Ala Lys Leu Gln Asp Ala Leu Gly
                85                  90                  95
Asp Arg Phe Leu Leu Val Thr Gln Asn Ile Asp Asn Leu His Glu Arg
            100                 105                 110
Ala Gly Asn Thr Asn Val Ile His Met His Gly Glu Leu Leu Lys Val
        115                 120                 125
Arg Cys Ser Gln Ser Gly Gln Val Leu Asp Trp Thr Gly Asp Val Thr
    130                 135                 140
Pro Glu Asp Lys Cys His Cys Cys Gln Phe Pro Ala Pro Leu Arg Pro
145                 150                 155                 160
His Val Val Trp Phe Gly Glu Met Pro Leu Gly Met Asp Glu Ile Tyr
                165                 170                 175
Met Ala Leu Ser Met Ala Asp Ile Phe Ile Ala Ile Gly Thr Ser Gly
            180                 185                 190
His Val Tyr Pro Ala Ala Gly Phe Val His Glu Ala Lys Leu His Gly
        195                 200                 205
Ala His Thr Val Glu Leu Asn Leu Glu Pro Ser Gln Val Gly Asn Glu
    210                 215                 220
Phe Ala Glu Lys Tyr Tyr Gly Pro Ala Ser Gln Val Val Pro Glu Phe
225                 230                 235                 240

Val Glu Lys Leu Leu Lys Gly Leu Lys Ala Gly Ser Ile Ala
                245                 250

<210> 4
<211> 240
<212> PRT
<213> Artificial Sequence

<220>
<223> Dealocase-1

<400> 4
Lys Pro Arg Val Leu Val Leu Thr Gly Ala Gly Ile Ser Ala Glu Ser
1               5                   10                  15
Gly Ile Arg Thr Phe Arg Ala Ala Asp Gly Leu Trp Glu Glu His Arg
            20                  25                  30
Val Glu Asp Val Ser Thr Pro Glu Gly Phe Asp Arg Asp Pro Glu Leu
        35                  40                  45
Val Gln Ala Phe Trp Asn Ala Trp Arg Arg Gln Leu Gln Gln Pro Glu
    50                  55                  60
Ile Gln Pro Asn Ala Ala His Leu Ala Leu Ala Lys Leu Gln Asp Ala
65                  70                  75                  80
Leu Gly Asp Arg Phe Leu Leu Val Thr Gln Asn Val Asp Asn Leu His
                85                  90                  95
Glu Arg Ala Gly Asn Thr Asn Val Ile His Met His Gly Glu Leu Leu
            100                 105                 110
Lys Val Arg Cys Ser Gln Ser Gly Gln Val Leu Asp Trp Thr Gly Asp
        115                 120                 125
Val Thr Pro Glu Asp Lys Cys His Cys Cys Gln Phe Pro Ala Pro Leu
    130                 135                 140
Arg Pro His Val Val Trp Phe Gly Glu Met Pro Leu Gly Met Asp Glu
145                 150                 155                 160
Ile Tyr Met Ala Leu Ser Met Ala Asp Ile Phe Ile Ala Ile Gly Thr
                165                 170                 175
Ser Gly His Val Tyr Pro Ala Ala Gly Phe Val His Glu Ala Lys Leu
            180                 185                 190
His Gly Ala His Thr Val Glu Leu Asn Leu Glu Pro Ser Gln Val Gly
        195                 200                 205
Asn Glu Phe Ala Glu Lys Tyr Tyr Gly Pro Ala Ser Gln Val Val Pro
    210                 215                 220
Glu Phe Val Glu Lys Leu Leu Lys Gly Leu Lys Ala Gly Ser Ile Ala
225                 230                 235                 240

<210> 5
<211> 240
<212> PRT
<213> Artificial Sequence

<220>
<223> Dealocase-2

<400> 5
Lys Pro Arg Val Leu Val Leu Thr Gly Ala Gly Ile Ser Ala Glu Ser
1               5                   10                  15
Gly Ile Arg Thr Phe Arg Ala Ala Asp Gly Leu Trp Glu Glu His Arg
            20                  25                  30
Val Glu Asp Val Gly Thr Pro Glu Gly Phe Asp Arg Asp Pro Glu Leu
        35                  40                  45
Val Gln Ala Phe Tyr Asn Ala Gly Arg Arg Gln Leu Gln Gln Pro Glu
    50                  55                  60
Ile Gln Pro Asn Ala Ala His Leu Ala Leu Ala Lys Leu Gln Asp Ala

65                  70                  75                  80
Leu Gly Asp Arg Phe Leu Leu Val Thr Gln Asn Val Asp Asn Leu His
                85                  90                  95
Glu Arg Ala Gly Asn Thr Asn Val Ile His Met His Gly Glu Leu Leu
            100                 105                 110
Lys Val Arg Cys Ser Gln Ser Gly Gln Val Leu Asp Trp Thr Gly Asp
        115                 120                 125
Val Thr Pro Glu Asp Lys Cys His Cys Cys Gln Phe Pro Ala Pro Leu
    130                 135                 140
Arg Pro His Val Val Trp Phe Gly Glu Met Pro Leu Gly Met Asp Glu
145                 150                 155                 160
Ile Tyr Met Ala Leu Ser Met Ala Asp Ile Phe Ile Ala Ile Gly Thr
                165                 170                 175
Ser Gly His Val Tyr Pro Ala Ala Gly Phe Val His Glu Ala Lys Leu
            180                 185                 190
His Gly Ala His Thr Val Glu Leu Asn Leu Glu Pro Ser Gln Val Gly
        195                 200                 205
Asn Glu Phe Ala Glu Lys Tyr Tyr Gly Pro Ala Ser Gln Val Val Pro
    210                 215                 220
Glu Phe Val Glu Lys Leu Leu Lys Gly Leu Lys Ala Gly Ser Ile Ala
225                 230                 235                 240

<210> 6
<211> 240
<212> PRT
<213> Artificial Sequence

<220>
<223> Dealocase-3

<400> 6
Lys Pro Arg Val Leu Val Leu Thr Gly Ala Gly Ile Ser Ala Glu Ser
1               5                   10                  15
Gly Ile Arg Thr Phe Arg Ala Ala Asp Gly Leu Trp Glu Glu His Arg
            20                  25                  30
Val Glu Asp Val Ala Thr Pro Glu Gly Phe Asp Arg Asp Pro Glu Leu
        35                  40                  45
Val Gln Ala Phe Tyr Asn Ala Arg Arg Arg Gln Leu Gln Gln Pro Glu
    50                  55                  60
Ile Gln Pro Asn Ala Ala His Leu Ala Leu Ala Lys Leu Gln Asp Ala
65                  70                  75                  80
Leu Gly Asp Arg Phe Leu Leu Val Thr Gln Asn Ser Asp Asn Leu His
                85                  90                  95
Glu Arg Ala Gly Asn Thr Asn Val Ile His Met His Gly Glu Leu Leu
            100                 105                 110
Lys Val Arg Cys Ser Gln Ser Gly Gln Val Leu Asp Trp Thr Gly Asp
        115                 120                 125
Val Thr Pro Glu Asp Lys Cys His Cys Cys Gln Phe Pro Ala Pro Leu
    130                 135                 140
Arg Pro His Leu Val Trp Phe Gly Glu Met Pro Leu Gly Met Asp Glu
145                 150                 155                 160
Ile Tyr Met Ala Leu Ser Met Ala Asp Ile Phe Ile Ala Ile Gly Thr
                165                 170                 175
Ser Gly His Val Tyr Pro Ala Ala Gly Phe Val His Glu Ala Lys Leu
            180                 185                 190
His Gly Ala His Thr Val Glu Leu Asn Leu Glu Pro Ser Gln Val Gly
        195                 200                 205
Asn Glu Phe Ala Glu Lys Tyr Tyr Gly Pro Ala Ser Gln Val Val Pro
    210                 215                 220
Glu Phe Val Glu Lys Leu Leu Lys Gly Leu Lys Ala Gly Ser Ile Ala
225                 230                 235                 240

<210> 7
<211> 240
<212> PRT
<213> Artificial Sequence

<220>
<223> Debocylase-1

<400> 7
Lys Pro Arg Val Leu Val Leu Thr Gly Ala Gly Ile Ser Ala Glu Ser
1               5                   10                  15
Gly Ile Arg Thr Phe Arg Ala Ala Asp Gly Leu Trp Glu Glu His Arg
            20                  25                  30
Val Glu Asp Val Ala Thr Pro Glu Gly Phe Asp Arg Asp Pro Glu Leu
        35                  40                  45
Val Gln Ala Phe Tyr Asn Ala Arg Arg Arg Gln Leu Gln Gln Pro Glu
    50                  55                  60
Ile Gln Pro Asn Ala Ala His Leu Ala Leu Ala Lys Leu Gln Asp Ala
65                  70                  75                  80
Leu Gly Asp Arg Phe Leu Leu Val Thr Gln Asn Val Asp Asn Leu His
                85                  90                  95
Glu Arg Ala Gly Asn Thr Asn Val Ile His Met His Gly Glu Leu Leu
            100                 105                 110
Lys Val Arg Cys Ser Gln Ser Gly Gln Val Leu Asp Trp Thr Gly Asp
        115                 120                 125
Val Thr Pro Glu Asp Lys Cys His Cys Cys Gln Phe Pro Ala Pro Leu
    130                 135                 140
Arg Pro His Leu Val Trp Phe Gly Glu Met Pro Leu Gly Met Asp Glu
145                 150                 155                 160
Ile Tyr Met Ala Leu Ser Met Ala Asp Ile Phe Ile Ala Ile Gly Thr
                165                 170                 175
Ser Gly His Val Tyr Pro Ala Ala Gly Phe Val His Glu Ala Lys Leu
            180                 185                 190
His Gly Ala His Thr Val Glu Leu Asn Leu Glu Pro Ser Gln Val Gly
        195                 200                 205
Asn Glu Phe Ala Glu Lys Tyr Tyr Gly Pro Ala Ser Gln Val Val Pro
    210                 215                 220
Glu Phe Val Glu Lys Leu Leu Lys Gly Leu Lys Ala Gly Ser Ile Ala
225                 230                 235                 240


<210> 8
<211> 240
<212> PRT
<213> Artificial Sequence

<220>
<223> Decrotonylase-1

<400> 8
Lys Pro Arg Val Leu Val Leu Thr Gly Ala Gly Ile Ser Ala Glu Ser
1               5                   10                  15
Gly Ile Arg Thr Phe Arg Ala Ala Asp Gly Leu Trp Glu Glu His Arg
            20                  25                  30
Val Glu Asp Val Arg Thr Pro Glu Gly Phe Asp Arg Asp Pro Glu Leu
        35                  40                  45
Val Gln Ala Phe Gly Asn Ala Thr Arg Arg Gln Leu Gln Gln Pro Glu
    50                  55                  60
Ile Gln Pro Asn Ala Ala His Leu Ala Leu Ala Lys Leu Gln Asp Ala

65                  70                  75                  80
Leu Gly Asp Arg Phe Leu Leu Val Thr Gln Asn Arg Asp Asn Leu His
                85                  90                  95
Glu Arg Ala Gly Asn Thr Asn Val Ile His Met His Gly Glu Leu Leu
            100                 105                 110
Lys Val Arg Cys Ser Gln Ser Gly Gln Val Leu Asp Trp Thr Gly Asp
        115                 120                 125
Val Thr Pro Glu Asp Lys Cys His Cys Cys Gln Phe Pro Ala Pro Leu
    130                 135                 140
Arg Pro His Leu Val Trp Phe Gly Glu Met Pro Leu Gly Met Asp Glu
145                 150                 155                 160
Ile Tyr Met Ala Leu Ser Met Ala Asp Ile Phe Ile Ala Ile Gly Thr
                165                 170                 175
Ser Gly His Val Tyr Pro Ala Ala Gly Phe Val His Glu Ala Lys Leu
            180                 185                 190
His Gly Ala His Thr Val Glu Leu Asn Leu Glu Pro Ser Gln Val Gly
        195                 200                 205
Asn Glu Phe Ala Glu Lys Tyr Tyr Gly Pro Ala Ser Gln Val Val Pro
    210                 215                 220
Glu Phe Val Glu Lys Leu Leu Lys Gly Leu Lys Ala Gly Ser Ile Ala
225                 230                 235                 240